# EUROPÄISCHE PATENTSCHRIFT

(11) **EP 3 124 004 B1**
(45) Veröffentlichungstag und Bekanntmachung des Hinweises auf die Patenterteilung: **18.03.2020**
(21) Anmeldenummer: 15178506.0
(22) Anmeldetag: 27.07.2015
(51) Int. Cl.: A61H 23/00, A61H 23/04, A61H 15/00, A61B 17/22, A61B 17/00

(54) **GERÄT ZUR BEHANDLUNG DES MENSCHLICHEN ODER TIERISCHEN KÖRPERS MIT MECHANISCHEN STÖSSEN**
DEVICE FOR TREATMENT OF THE HUMAN OR ANIMAL BODY WITH MECHANICAL IMPACTS
APPAREIL DE TRAITEMENT DU CORPS HUMAIN OU ANIMAL A L'AIDE DE CHOCS MÉCANIQUES

(43) Veröffentlichungstag der Anmeldung: 01.02.2017
(73) Patentinhaber: STORZ MEDICAL AG, 8274 Tägerwilen (CH)
(72) Erfinder: NOVAK, Pavel, 8234 Stetten (CH); SCHULZ, Johannes Manfred, 8274 Tägerwilen (CH); SWART, Stephan Gerhard, 47447 Moers (DE); DI MAIO, Carlo, 47199 Duisburg (DE)
(74) Vertreter: König Szynka Tilmann von Renesse Patentanwälte Partnerschaft mbB

(56) Entgegenhaltungen:
- EP-A1- 2 181 730
- WO-A1-2013/107898
- DE-C- 589 285
- DE-U1- 29 512 015
- DE-U1-202010 007 860
- US-A1- 2002 107 459
- US-A1- 2002 177 795
- US-A1- 2003 009 116

## Beschreibung

Die vorliegende Erfindung bezieht sich auf ein Gerät zur Behandlung des menschlichen oder tierischen Körpers durch Ausüben von Stößen auf die Körperoberfläche. Im Folgenden wird zur Vereinfachung vom Körper eines Patienten gesprochen, der bevorzugt menschlich ist.

Im Stand der Technik sind verschiedene Geräte des beschriebenen Grundtyps bekannt. Die DE 197 25 477 C beschreibt bspw. ein solches Gerät, bei dem durch die Kollision eines pneumatisch beschleunigten Schlagteils oder Projektils mit einem zunächst ruhenden Prallkörper oder Applikator eine Druckwelle ausgelöst wird, die dadurch in den Körper des Patienten eingekoppelt werden kann, dass eine Applikatorfrontfläche zum Zeitpunkt der Kollision auf den Patientenkörper aufgelegt wird. Dieser Gerätetyp leitet sich in seiner Entstehungsgeschichte von Lithotripsiegeräten ab, bei denen solche Druckwellen z. B. über eine lange stabförmige Sonde an der Frontfläche des Applikators auf einen Nierenstein oder Ähnliches übertragen werden können, um diesen zu desintegrieren.

Der Schwerpunkt bei der Darstellung liegt in jedem Fall auf der durch die Kollision erzeugten Druckwelle, die mehr oder weniger einer eigentlichen Stoßwelle ähneln soll, wie sie klassische, in der Regel fokussierende Lithotripsiegeräte z. B. mit piezoelektrischen oder elektromagnetischen Aktuatoren und Fokussierung auf einen Stein erzeugen. Solche Druckwellen können Anstiegsflanken mit einer Breite im Bereich weniger µs und einer Amplitude im niedrigen zweistelligen MPa-Bereich haben (z. B. 2 µs und 15 MPa gemessen 1 cm vor der Frontfläche). In dem zitierten Dokument wird hingegen betont, dass die physikalisch an sich unvermeidliche makroskopische Schwerpunktbewegung des Applikators möglichst klein gehalten werden soll, weil sie als störend angesehen wird.

Als zweites Beispiel wird auf die DE 20 2004 011 323 U und, mit sehr ähnlichem Inhalt, die US 2011/0054367 A1 verwiesen. Dort wird ein hinsichtlich des technischen Aufbaus ähnliches Gerät beschrieben, bei dem allerdings u. a. die elastische Aufhängung des Applikators im Gehäuse auf größere Schwerpunktsbewegungen des Applikators ("Hübe") ausgelegt ist. Dort wird betont, dass die therapeutische Wirkung durchaus auch, oder vorwiegend in den eigentlichen makroskopischen Stößen (also infolge des Hubes) gesehen werden kann, was auch von der Indikation abhängt.

Die vorliegende Erfindung richtet sich allgemein auf Geräte dieses Bautyps, und zwar sowohl hinsichtlich der Anwendung von Druckwellen als auch hinsichtlich der Anwendung "makroskopischer Stöße" des Applikator auf die Körperoberfläche.

Die Merkmalskombination des Oberbegriffs des Anspruchs 1 ergibt sich aus der DE 589 285 C mit einem Rollmassageapparat, der elektromagnetisch einen federnd aufgehängten Anker in eine Resonanzbewegung quer zur Gehäuselängsrichtung bringt.

Sie ergeben sich auch aus der WO 2013/107 898 A1 mit einem pistolenartig aufgebauten Massagegerät, dessen Massagekopf periodische oszillierende Bewegungen mit asymmetrischer Schwingungsform, aber ohne zugrundeliegende Kollisionen, vollführt.

Schließlich ergeben sich diese Merkmale in Kombination auch aus der US 2002/0107459 A1 mit einem weiteren vibrierenden Massagegerät, das austauschbare Massageköpfe unterschiedlicher Formen zeigt.

Schließlich wird verwiesen auf die US 2002/0177795 A1 mit einem Gerät zur Erzeugung von kollisionsbedingten Stößen zur chiropraktischen Wirbelsäulenbehandlung. Dabei ist auf einen von diesen Stößen beaufschlagten Applikator ein stumpfkonischer Schaumstoffkopf aufgesetzt.

Dabei liegt der Erfindung die Aufgabe zugrunde, ein solches Gerät hinsichtlich weitergehender Anwendungsmöglichkeiten auf der Patientenkörperoberfläche weiterzubilden.

Die Aufgabe wird gelöst durch ein Gerät nach Anspruch 1. Erfindungsgemäß ist dabei ein drehbares Applikatorteil vorgesehen. Es gibt also ein Drehlager, also insbesondere ein Wellenlager mit einem Achsstift aber z. B. auch ein Kugellager (Kugel geführt in Pfanne) oder ein komplexeres Lager wie z. B. ein Kardanlager. Über dieses Lager ist das drehbare Applikatorteil relativ zu dem restlichen Gerät um zumindest eine Drehachse drehbar, wobei diese Drehachse nicht parallel, also gewinkelt, zu der Richtung der von dem Gerät auszuführenden Stöße liegt. Vorzugsweise liegt die Drehachse senkrecht zur Stoßrichtung.

Damit kann das Applikatorteil beim Aufdrücken auf die zu behandelnde Körperoberfläche abrollen. Dementsprechend soll das drehbare Applikatorteil in solcher Weise zugänglich liegen, dass das Gerät in der entsprechenden Weise auf den Körper aufgesetzt werden kann. In anderen Worten soll das drehbare Applikatorteil exponiert liegen. Bevorzugt ist eine Anordnung an dem distalen Ende eines entsprechenden Handstücks, das am anderen (proximalen) Ende über eine Leitung von einem Basisgerät versorgt ist.

Die Erfinder haben nämlich festgestellt, dass es praktisch sein kann, den Applikator z. B. in Bezug auf die Stoßrichtung schräg auf die Patientenkörperoberfläche aufzusetzen und dann auf der Körperoberfläche zu bewegen. Insbesondere können dabei Gewebepartien unter der Haut parallel massiert und/oder gewissermaßen vor dem Gerät hergeschoben werden, wobei sie gleichzeitig mit den Stößen und (in einem von Fall zu Fall unterschiedlichen Maß) Druckwellen beaufschlagt werden. Die konventionell bekannten Geräte mit feststehenden und mehr oder weniger flachen Frontflächen der Applikatoren sind dazu relativ schlecht geeignet, weil sie auf der Hautoberfläche nur bei mehr oder weniger senkrechter Stellung des Geräts relativ zur Körperoberfläche gehalten werden können und in dieser Haltung schlecht verschoben werden können.

Im Unterschied dazu lässt sich das erfindungsgemäße Gerät über die Oberfläche rollen und damit leicht und schonend bewegen, auch wenn ein gewisser Anpressdruck ausgeübt wird.

Der Mechanismus zum Erzeugen der Stöße des Applikators ist in diesem Zusammenhang in unterschiedlichster Ausführung denkbar, insbesondere auch durch eine direkte Beaufschlagung des Applikators mittels eines elektromagnetischen Mechanismus oder auch eines Druckfluidpulses. Bevorzugt ist aber die aus den zitierten Dokumenten bereits bekannte Verwendung eines beschleunigten Projektils zur Kollision mit dem Applikator, womit relativ heftige und (im Sinn der Applikatorgeschwindigkeit) schnelle Stöße realisiert werden können und auch die gleichzeitige oder sogar schwerpunktmäßige Verwendung von Druckwellen möglich ist. Das Projektil wiederum lässt sich ebenfalls in unterschiedlicher Weise beschleunigen, insbesondere auch elektromagnetisch, wobei auch hier eine pneumatische Beaufschlagung des Projektils gemäß den zitierten Schriften bevorzugt ist.

Der Begriff "Applikator" bezeichnet hier übrigens auch abgesehen von der Drehbarkeit nicht zwingend ein einstückiges Teil. Es ist denkbar und anwendungsabhängig auch bevorzugt, dass das Projektil z. B. auf ein erstes Teil schlägt, das den Stoß und/oder die Druckwelle auf ein zweites Applikatorteil überträgt, das seinerseits mit der Haut des Patienten in Kontakt steht. Im Stand der Technik ist auch gelegentlich von einem Zwischenstück zwischen Applikator und Projektil die Rede; hier wird dann von einem mehrteiligen Applikator gesprochen, wobei die beiden Applikatorteile fest verbunden sein können, aber nicht sein müssen.

Wie eingangs bereits erwähnt, kann ein relativ großer Hub des Applikators durchaus gewünscht sein, wobei im vorliegenden Zusammenhang das Gerät vorzugsweise so ausgelegt ist, dass Hübe über 1 mm erreichbar sind, wie schon in der US 2011/0054367 A1 ausgeführt.

Das drehbare Applikatorteil ist vorzugsweise rotationssymmetrisch in Bezug auf die Drehachse als Symmetrieachse, um sich besonders gut abrollen zu lassen. Diese Rotationssymmetrie betrifft dabei die grobe Gestalt, also sozusagen eine Einhüllende, und steht oberflächlichen Strukturen, etwa Rillen zu Massagezwecken, nicht entgegen. Das drehbare Applikatorteil ist ferner vorzugsweise spiegelsymmetrisch, und zwar in Bezug auf eine Symmetrieebene senkrecht zur Drehachse. Das erleichtert im Regelfall die Anwendung und erlaubt bei geeigneter Positionierung des drehbaren Applikatorteils relativ zu dem restlichen Gerät oder Handstück auch eine entsprechende Benutzung "von zwei Seiten".

Die drehbare Lagerung erfolgt vorzugsweise beidseits, insbesondere mit einem durchgehenden Lagerstift. Eine solche Konstruktion ist einfach und gleichzeitig stabil.

Ferner sind abgerundete Kanten bevorzugt, und zwar soweit Berührungsmöglichkeiten mit der zu behandelnden Körperoberfläche bestehen, also außenseitig. Nicht betroffen sind also z. B. Kanten am Übergang zu einer zentrischen Bohrung für den Lagerstift.

Eine bevorzugte Form ist eine Tonnenform, also eine im Wesentlichen zylindrische Form mit allerdings verdicktem Mittenbereich, also einem konvexen Außenprofil bezogen auf eine die Drehachse enthaltende Schnittebene. Hierzu wird auf das erste Ausführungsbeispiel verwiesen. Mit einer solchen Form lassen sich einerseits großflächige Behandlungen erreichen, kann aber andererseits im bezüglich der Drehachse mittleren Bereich ein etwas höherer Anpressdruck und damit eine konzentrierte Wirkung erzielt werden.

Eine andere bevorzugt Form weist mindestens zwei in Bezug auf die Drehachse radial vorstehende Bereiche auf, die jeweils zum Kontakt mit der Körperoberfläche ausgelegt sind. Hierzu wird auf das zweite Ausführungsbeispiel verwiesen. Eine solche Form kann z. B. günstig sein, um beidseits neben einem nicht zu behandelnden oder zu schonenden Bereich entlang fahren zu können, bspw. rechts und links neben der Wirbelsäule eines Patienten.

Vorbekannte Applikatoren bestanden im Regelfall aus rostfreiem Stahl. Im vorliegenden Fall kommen grundsätzlich auch Metalle, so auch rostfreier Stahl, in Betracht. Daneben sind als metallische Materialien Aluminium und Titan zu nennen, wobei beide eine relativ geringe Massendichte haben und damit relativ leichte Applikatoren ermöglichen. Das kann den Vorteil haben, bei gegebener Geometrie eine stärkere Beschleunigung und damit auch größere Hubauslenkung des Applikators zu ermöglichen, was im vorliegenden Fall gewünscht sein kann. Titan zeichnet sich zudem durch eine besonders hohe mechanische Belastbarkeit aus, eignet sich also besonders für Anwendungen mit vergleichsweise hohen Projektilgeschwindigkeiten und/oder Projektilmassen. Da Titan außerdem eine besonders gute physiologische Verträglichkeit auszeichnet, kann es als Applikatormaterial auch unabhängig von der Belastbarkeit von Vorteil sein.

In Betracht kommen, insbesondere für das drehbare Applikatorteil, ferner Keramiken, vgl. Anmeldenummer 08 003 840.9 / EP 2 095 843, und Kunststoffe. Im Vergleich zu rostfreiem Stahl weisen auch diese den Vorteil geringer Massendichte auf. Sie haben ferner eine geringere Wärmeleitfähigkeit als Metalle, sodass der Patient den Applikator subjektiv als wärmer empfindet. Das gilt vor allem für Kunststoffe, wieder insbesondere für das drehbaren Teil. Kunststoffe kommen insbesondere auch dann in Betracht, wenn auf die Einkopplung einer Druckwelle geringerer Wert gelegt wird, weil, jedenfalls bei einer größeren Stärke des Kunststoffs, im Vergleich zu den vorgenannten Materialien etwas größere Wellenleitungsverluste zu erwarten sind. Gleiche Argumente gelten für Holz.

Nach einer weiteren Ausgestaltung ist der Applikator in einer lösbaren Kupplung gehalten, sodass zumindest ein distales Applikatorteil (mit dem drehbaren Applikatorteil) aus dem Gerät nach vorn herausgezogen werden kann. Zur Klarstellung: Die Begriffe "proximal" und "distal" werden hier aus der Perspektive eines Benutzers des Geräts gebraucht; in anderen Worten bedeutet "distal" eine dem Patienten zugewandte Position und "proximal" eine dazu entgegengesetzte. Im gleichen Sinn wird als "vorn" die distale Richtung bezeichnet, also auf den Patienten zu.

Die Erfinder haben festgestellt, dass die Optimierung der Applikatoreigenschaften im Hinblick auf bestimmte Behandlungen, bspw. der Wechsel zwischen einem erfindungsgemäßen Applikator und einem konventionellen oder zwischen verschiedenen Applikatoren mit drehbaren Applikatorteilen, vorteilhafterweise mit im Übrigen unverändertem Gerät von Fall zu Fall vorgenommen werden kann.

Ein Applikator in diesem Sinn kann mehrteilig aufgebaut sein, wobei die Entnehmbarkeit primär einen distalen Teil betrifft. Dieser distale Teil des Applikators kann dann gegen andere entsprechende distale Applikatorteile getauscht werden, zum Beispiel weil das Applikatorteil nur eine begrenzte Lebensdauer hat, weil es gereinigt oder sterilisiert werden soll oder weil ein anderer Typ dieses distalen Applikatorteils (oder des gesamten Applikators) verwendet werden soll. Insbesondere ist es möglich, in ihrem distalen Teil unterschiedlich geformte drehbare Applikatorteile, möglicherweise auch aus verschiedenen Materialien, einzusetzen.

Der Austausch erfolgt dabei sehr einfach und schnell, vorzugsweise völlig werkzeuglos und ebenfalls vorzugsweise ohne Abbauen eines weiteren Geräteteils, etwa eine Aufschraubkappe bei konventionellen Geräten dieses Typs. Bei diesen konventionellen Geräten war der Applikator in der Regel einteilig und innerhalb des Gerätegehäuses etwas dicker (senkrecht zur Stoßrichtung) als eine vordere Öffnung. Ein Ausbau war daher möglich, aber nur nach Teilzerlegung des Gehäuses, nämlich Abschrauben einer Schraubkappe und nicht durch Lösen einer für den Applikatorwechsel vorgesehenen Kupplung.

Eine bevorzugte Ausführungsform sieht eine Verdrehsicherung des Applikators in dem Gerät vor (nicht zwingend in der Kupplung). Dementsprechend kann zum Beispiel die Kupplung selbst eine Verdrehsicherheit herstellen oder durch Einschieben eines entsprechend gestalteten Bereichs des Applikators in eine entsprechende Aufnahme eine Verdrehsicherheit hergestellt werden, besonders bevorzugterweise durch einen Mehrkantstift auf der Applikatorseite und eine dazu passende Aufnahme auf der Geräteseite, etwa durch einen hexagonalen Formschluss. Dies beinhaltet Varianten, bei denen die Verdrehsicherung erst durch einen axial gerichteten Druck entsteht und ohne diesen Andruck eine Drehbarkeit gegeben ist. Die Verdrehsicherung betrifft dabei bevorzugt zumindest auch eine von der (die Bewegung in Stoßrichtung betreffende) Kupplung selbst unabhängige Aufnahme, um die Möglichkeiten zur Kraftaufnahme zu verbessern. Zur Veranschaulichung wird auf das Ausführungsbeispiel verwiesen.

Die Kupplung kann vorzugsweise ebenfalls mit einem Formschluss arbeiten, der nicht zwingend mit dem Formschluss für die Verdrehsicherung übereinstimmen muss. Insbesondere kann eine Klemmhülse vorgesehen sein, die (vorzugsweise in Stoßrichtung) verschiebbar ist und dabei zumindest ein Formschlusselement in eine entsprechende Aufnahme an dem Applikator hereindrückt bzw. beim Lösen freigibt. Insbesondere kann ein Gehäuseteil des Geräts gegen eine Federkraft verschiebbar sein und dabei die Klemmhülse aufweisen oder mitverschieben. Als Formschlusselement kommt eine Kugel in Betracht, wobei vorzugsweise mindestens zwei Kugeln vorgesehen sind. Die Kugeln oder andere Formschlusselemente können durch eine schräge Fläche an einer Innenseite der Klemmhülse beaufschlagt werden.

Im Folgenden wird die Erfindung anhand eines Ausführungsbeispiels beschrieben, wobei die Offenbarung im Ausführungsbeispiel und auch im vorstehenden Text nicht auf die jeweils dargestellten Merkmalskombinationen beschränkt sein soll, sondern einzelne Merkmale auch in anderem Zusammenhang oder für sich wichtig sein können.

Figur 1 zeigt einen Längsschnitt durch einen vorderen Gehäuseteil eines erfindungsgemäßen Geräts mit einer Applikatorkupplung.

Die Figuren 2-4 zeigen erfindungsgemäße Varianten des Applikators.

Der dem Ausführungsbeispiel zugrunde liegende Typ eines pneumatisch betriebenen Druckwellengeräts ist hinlänglich bekannt, wozu auf den eingangs zitierten Stand der Technik verwiesen werden kann. Er weist eine Basisstation mit einer Pneumatikversorgung, eine davon ausgehende Versorgungsleitung und am Ende der Versorgungsleitung ein mobiles und mit der Hand handhabbares Handstück auf, das hier als das Gerät bezeichnet wird. Die Pneumatikversorgung ist über die Leitung und ein entsprechendes Schaltventil an ein Beschleunigungsrohr angeschlossen, dessen in der Darstellung der Figur linkes Ende in der Figur eingezeichnet und mit 1 beziffert ist. Der Pneumatikanschluss findet sich am nicht dargestellten rechten Ende desselben Rohres 1 und in dem Rohr 1 ist ein nicht dargestelltes und den Querschnitt des Rohres ausfüllendes Projektil bewegbar, das am Ende seiner Bewegungsstrecke auf die rechte Stirnfläche eines proximalen Applikatorteils 2 aufprallen kann.

Dieses proximale Applikatorteil 2 hat eine im Vergleich zum Stand der Technik weitgehend konventionelle Form und stützt sich mit einem radial vergrößerten Flansch und dessen distaler Schulterseite an einem Elastomerschlauchstück 3 ab, das bei den Kollisionen verformbar ist und eine Bewegung des proximalen Applikatorteils 2 im Bereich über einem Millimeter zulassen kann. Es ist ferner für die Rückstellung verantwortlich und entkoppelt die Schläge vom Gerätegehäuse. An der distalen Seite läuft dieses proximale Applikatorteil 2 in einem zylindrischen Stift aus, der eine Führungsmanschette 4 durchsetzt und zur distalen Seite etwas darüber hinausragt. Bei konventionellen Geräten wäre die distale Stirnfläche dieses Stifts die Kontaktfläche des Applikators zum Patienten und die Führungsmanschette 4 in einer (oder als) abschraubbaren Kappe realisiert. Man müsste also die Kappe abnehmen und damit eine Teilzerlegung vornehmen, bevor man den Applikator (in diesem Fall das proximale Applikatorteil 2) entnehmen kann.

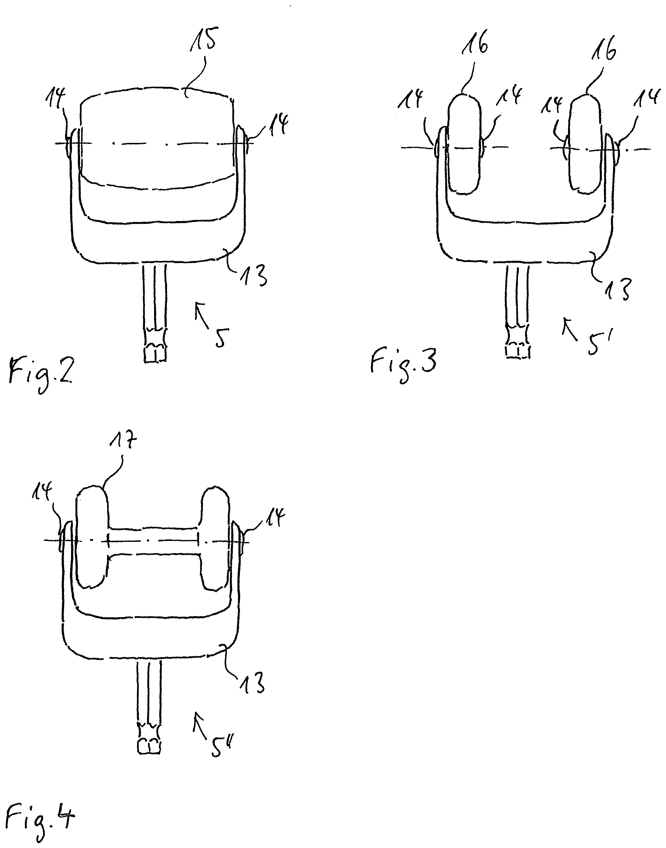

Die Figur zeigt ferner, dass sich die linke Stirnfläche des proximalen Applikatorteils 2 in Kontakt mit der rechten Stirnfläche eines distalen Applikatorteils 5 befindet. Auf dieses distale Applikatorteil 5 wird anhand der Figuren 2-4 näher eingegangen. Es ist in einem Aufnahmestück 6 geführt, und zwar formschlüssig, z. B hexagonal, und damit gegenüber diesem Aufnahmestück 6 gegen Verdrehung gesichert. Das Aufnahmestück 6 seinerseits muss entsprechend fest verdrehgesichert im übrigen Gehäuse gehalten sein, wozu entsprechende in der Figur nach rechts weisende Stirnflächen oder nach außen weisende Mantelflächen in reibungssteigernder Form beschichtet oder oberflächenstrukturiert sein können. Das kann auch so geschehen, dass ohne axialen Andruck noch eine Verdrehbarkeit gewährleistet ist, etwa um den Applikator nach Wunsch auszurichten, und nach Auflegen und Andrücken auf den Patientenkörper, also mit axialem Andruck, ein ausreichender Reibungsschluss zur Verdrehsicherung hergestellt wird. Um ein Fressen zwischen dem distalen Applikatorteil 5 und dem Aufnahmestück 6 zu verhindern, können entsprechende Materialpaarungen verwendet werden, zum Beispiel applikatorseitig rostfreier Edelstahl und aufnahmeseitig Bronze, hochfester Kunststoff (zum Beispiel PEEK) oder Keramik.

Die Figur zeigt ferner, dass in dem Aufnahmestück 6 (in der Figur oben und unten) Öffnungen für Formschlusskugeln 7 vorgesehen sind, die nach innen in entsprechende außenseitige Nuten an dem distalen Applikatorteil 5 eingreifen. Diese Nuten sind in der Figur nach rechts länger als nötig, sodass sich der gesamte Applikator 5 und 6 bei einem Stoß nach links bewegen kann. Andererseits verhindert der Formschluss zwischen den Kugeln 7 und den dazugehörenden Nuten ein Herausfallen oder -schießen des distalen Applikatorteils aus dem Gehäuse. Das distale Applikatorteil kann vielmehr zum Beispiel einfach durch Aufdrücken auf dem Patienten nach einem Stoß wieder in seine gezeichnete Ursprungslage zurückgeschoben werden.

Radial außerhalb der Kugeln 7 erkennt man eine Klemmhülse 8, die ersichtlich an ihrem linken Ende eine innere Schrägfläche aufweist. Wenn diese Klemmhülse 8 aus der gezeichneten Position heraus nach rechts verschoben wird, können die Kugeln 7 radial nach außen weichen und kann das distale Applikatorteil 5 insgesamt einfach nach links aus dem Gehäuse herausgezogen werden. Für dieses Verschieben der Klemmhülse 8 sorgt ein händisches (nach rechts) Hineindrücken eines von außen zugänglichen Gehäuseteils 9, wobei eine Schraubenfeder 10 dagegen drückt bzw. für die Rückführbewegung sorgt und dieses verschiebbare Gehäuseteil 9 in einem weiteren Teil 11 des Gehäuses verschiebbar und durch einen O-Ring gehemmt geführt ist. Dieses Teil 11 umgreift und hält auch das bereits erwähnte Aufnahmestück 6.

Die Kugeln 7 sind übrigens in radialer Richtung nicht kraftbeaufschlagt an sich, sondern werden verdrängt, wenn der Benutzer das distale Applikatorteil 5 herauszieht. Da es sich nur um zwei (oder in anderen Fällen um zum Beispiel drei, vier oder sechs) Kugeln handelt, für die jeweils eigene Nuten vorgesehen sind, tragen sie auch zur Verdrehsicherung bei; wesentlich für die Verdrehsicherung ist aber die beschriebene Mehrkant-Formschluss-Ausführung der Teile 5a außen und 6 innen.

In der Figur links von dem verschiebbaren und zur Betätigung der Kupplung dienenden Gehäuseteil 9 findet sich noch eine Sicherungsmutter 12, die auf dem Aufnahmeteil 6 aufgeschraubt und gegenüber diesem über einen O-Ring und gegenüber dem Teil 5 über einen Elastomerflachring abgedichtet ist. Dabei ist die Anlage zwischen dem Elastomerflachring (an dessen Innenrand) und dem hier hexagonalen ersten Stück des distalen Applikatorteils 5 gleitfähig.

Insgesamt erkennt man, dass ein Projektilstoß den gesamten Applikator nach links versetzt und dabei eine entsprechende Stoßbewegung des nicht gezeichneten distalsten Endes des Applikators (ganz links) gegen den Patientenkörper zur Folge hat, wobei die Einkopplung einer eigentlichen Druckwelle parallel auftreten wird, aber in vielen Fällen gar nicht wesentlich ist. Die Druckwelle wird dabei übrigens in dem proximalen Applikatorteil 2 erzeugt und über dieses und das distale Applikatorteil 5 in den Körper übertragen. Nach dem Stoß kehrt das proximale Applikatorteil 2 infolge der Kraftbeaufschlagung durch das Elastomerschlauchstück 3 selbsttätig nach rechts zurück; für das distale Applikatorteil 5 folgt dies aus der Druckauflage auf den Patientenkörper.

Wenn das distale Applikatorteil 5 gewechselt werden soll, schiebt der Benutzer das verschiebbare Gehäuseteil 9 nach rechts und damit die Klemmhülse 4 ebenfalls, sodass die Kugeln 7 radial nach außen gelangen können. Dabei sind übrigens die Öffnungen in dem Aufnahmestück 6 für die Kugeln 7 radial innen zu eng, sodass die Kugeln nicht herausfallen können. Beim Wiedereinsetzen eines anderen entsprechenden distalen Applikatorteils muss wieder das Gehäuseteil 9 nach rechts geschoben werden, um die Kugeln nach außen verdrängen zu können. Wenn das distale Applikatorteil 5 vollständig eingesetzt ist, kann man das Gehäuseteil 9 loslassen und verriegelt die Kupplung.

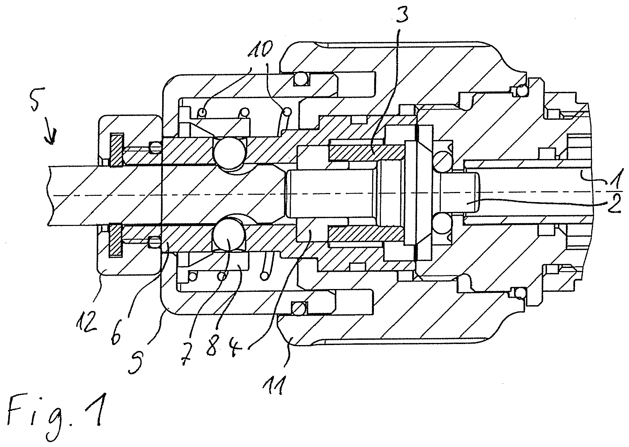

Die Figuren 2-4 zeigen die Ausgestaltung des Applikators bzw. konkret des distalen Applikatorteils 5 in weiteren Einzelheiten. Zunächst erkennt man in allen Figuren nach unten weisend den jeweiligen, hier hexagonalen Stab zur Aufnahme in dem Aufnahmestück 6 mit den außenseitigen Nuten für die Formschlusskugeln 7. Dieser Stab führt zu einer gabelartigen Verbreiterung 13, die in allen drei Varianten der Figuren 2-4 identisch ist und an ihren in Stoßrichtung (in den Figuren 2-4 nach oben und in Figur 1 nach links) weisenden Enden bzw. Gabelzinken Aufnahmen für Lagerstifte trägt.

Bei der Variante aus Figur 2 ist ein durchgängiger und die strichpunktierte Drehachse definierender Lagerstift vorgesehen, dessen größere abgeflachte Enden 14 erkennbar sind; das Gleiche gilt für Figur 4. In Figur 3 sind es zwei Lagerstifte mit ebenfalls abgeflachten Enden 14, und zwar jeweils ein Lagerstift pro Gabelarm. An den Lagerstiften gehalten sind drehbare Applikatorteile, und zwar ein durchgehendes konvex-tonnenförmiges drehbares Applikatorteil 15 in Figur 2, zwei jeweils radartig und mit konvexer Außenfläche versehene drehbare Applikatorteile 16 in Figur 3 und ein hantelartiges drehbares Applikatorteil 17 in Figur 4, das ähnlich den beiden Applikatorteilen 16 aus Figur 3, aber mit einer radial viel kleineren Verbindung ausgestaltet ist.

Nach dem Einsetzen in die Aufnahmekupplung aus Figur 1 übertragen sich die Stöße infolge der beschriebenen Kollisionen über die hexagonalen Stifte und die Gabeln 13 sowie die Lagerstifte auf die drehbaren Applikatorteile 15-17 und können von diesen in einen Patientenkörper eingekoppelt werden. Dazu wird das sich in Figur 1 rechts an den gezeichneten Bereich anschließende Handstück des Geräts (in einer weiteren Ausgestaltung gemäß dem eingangs zitierten Stand der Technik) so mit der Hand gehalten, dass die drehbaren Applikatorteile 15-17 auf der Körperoberfläche aufliegen. Dazu können die drehbaren Applikatorteile 15-17, z. B. zur Behandlung von Faszien, entlang der Haut geschoben werden und die darunterliegenden Faszien quasi vor sich herschieben, wobei die Drehbarkeit die Belastung für die Haut reduziert und die Tätigkeit des Therapeuten erleichtert. Dabei ist auch (aber nicht zwingend) eine schräge Haltung des Handstücks zur Körperoberfläche möglich (z. B. um die jeweilige Drehachse geneigt, sodass beim Schieben das drehbare Applikatorteil vor dem Handstück rollt), etwa mit Drehwinkel zwischen 30 % und 60 %. Ein Therapeut kann in dieser Form entlang der Körperoberfläche nach sogenannten Triggerpunkten suchen und Behandlungen mit der kombinierten Wirkung der beschriebenen Druckwellen, der eigentlichen Stöße des Geräts und der Massagewirkung ausführen.

Für verschiedene Anwirkungen lassen sich die vorderen Applikatorteile gemäß den Figuren 2-4 untereinander vertauschen, z. B. um mit den Beispielen aus den Figuren 3 und 4 entlang der Wirbelsäule so zu arbeiten, dass die vorstehenden Bereiche der drehbaren Applikatorteile 16 und 17 beidseits der Wirbelsäule abrollen. Mit Hilfe der verschiedenen Aufnahmekupplungen können die Teile gemäß den Figuren 2-4 auch gegen konventionelle oder anders geformte Applikatorteile ausgetauscht werden.

Bei den Ausführungsbeispielen bestehen die Gabel und der hexagonale Aufnahmestift aus Metall, z. B. rostfreiem Stahl oder Aluminium, was auch für die Lagerstifte gilt. Die drehbaren Applikatorteile 15-17 können aus den im Übrigen erwähnten Materialien, bspw. auch aus Kunststoff, bestehen.

## Patentansprüche

1. Gerät zur Behandlung des menschlichen oder tierischen Körpers mit
- einem Applikator (2, 5, 5', 5") zum Auflegen auf den Körper von au-βen,
- einem Gehäuse (9, 11, 12), in dem der Applikator (2, 5, 5', 5") gehalten ist, und
- einem Mechanismus zum Erzeugen von Stößen des Applikators (2, 5, 5', 5") relativ zu dem Gehäuse (9, 11, 12) in einer Stoßrichtung, sodass die Stöße bei dem Auflegen in den Körper eingekoppelt werden können,
wobei der Applikator (2, 5, 5', 5") ein Drehlager (14) aufweist, welches ein auf dem zu behandelnden Körper anzuordnendes drehbares Applikatorteil (15, 16, 17) des Applikators (2, 5, 5', 5") drehbar hält, und
eine Drehung des drehbaren Applikatorteils (15, 16, 17) um eine zu der Stoßrichtung gewinkelte Drehachse möglich ist,
**dadurch gekennzeichnet, dass** der Mechanismus zum Erzeugen der Stöße ein Projektil und eine pneumatische Einrichtung zum Beschleunigen des Projektils in solcher Weise aufweist, dass das Projektil auf den Applikator (2, 5, 5', 5") schlägt und dadurch den Stoß erzeugt,
und wobei der Applikator (2, 5, 5', 5") in dem Gerät durch einen Formschluss verdrehsicher gehalten wird, und zwar bezüglich Rotationen um eine zur Stoßrichtung parallele Achse.

2. Gerät nach Anspruch 1, bei dem die Drehachse senkrecht zu der Stoßrichtung liegt.

3. Gerät nach Anspruch 1 oder 2, bei dem das drehbare Applikatorteil (15, 16, 17) in Bezug auf die Drehachse rotationssymmetrisch ist.

4. Gerät nach einem der vorstehenden Ansprüche, bei dem das drehbare Applikatorteil (15, 16, 17) in Bezug auf eine zu der Drehachse senkrechte Ebene spiegelsymmetrisch ist.

5. Gerät nach einem der vorstehenden Ansprüche, bei dem das drehbare Applikatorteil (15, 17) an zwei entgegengesetzten Seiten drehbar gehalten ist, insbesondere von einer die Drehachse definierenden Achsstange durchsetzt ist.

6. Gerät nach einem der vorstehenden Ansprüche, bei dem das drehbare Applikatorteil (15, 16, 17) außenseitig abgerundete Kanten mit einem Krümmungsradius von mindestens 1mm aufweist.

7. Gerät nach Anspruch 3, auch in Verbindung mit einem der Ansprüche 4-6, bei dem das drehbare Applikatorteil (15) eine zylindrische Tonnenform mit einem konvexen Außenprofil in einer die Drehachse enthaltenden Schnittebene aufweist.

8. Gerät nach einem der Ansprüche 1-6, bei dem das drehbare Applikatorteil (17) mindestens zwei in Bezug auf die Drehachse radial vorstehende und zum Kontakt mit dem zu behandelnden Körper ausgelegte Bereiche aufweist.

9. Gerät nach einem der vorstehenden Ansprüche, bei dem der Applikator (2, 5, 5', 5") in seinem zum Auflegen ausgelegten drehbaren Teil (15, 16, 17) besteht aus Metall, insbesondere Aluminium oder Titan, Kunststoff, Keramik oder Holz.

10. Gerät nach einem der vorstehenden Ansprüche, das ausgelegt ist für einen Hub des Stoßes in der Stoßrichtung relativ zu dem Gehäuse (9, 11, 12) von mindestens 1 mm.

11. Gerät nach einem der vorstehenden Ansprüche, bei dem der Applikator (2, 5, 5', 5") in dem Gehäuse (9, 11, 12) in einer Kupplung (7, 8, 9, 10) gehalten ist, wobei zumindest ein distales Applikatorteil (5) mit dem drehbaren Applikatorteil (15, 16, 17) nach Lösen der Kupplung (7, 8, 9, 10) in distaler Richtung aus dem Gehäuse (9, 11, 12) herausziehbar ist.

12. Gerät nach einem der vorstehenden Ansprüche, bei dem der Applikator (2, 5, 5', 5") zumindest zweiteilig ist, wobei ein distales Applikatorteil (5, 5', 5") nach Lösen einer Kupplung (7, 8, 9, 10) herausziehbar ist und ein proximales Applikatorteil (2) in dem Gehäuse verbleibt und wobei das proximale Applikatorteil (2) den Stoß auf das distale Applikatorteil (5, 5', 5") überträgt.

## Claims

1. A device for the treatment of the human or animal body, comprising
- an applicator (2, 5, 5', 5") to be applied externally onto the body,
- a housing (9, 11, 12) in which the applicator (2, 5, 5', 5") is held, and
- a mechanism for generating impacts of the applicator (2, 5, 5', 5") relative to the housing (9, 11, 12) in a direction of impact so that the impacts can be coupled into the body when the applicator is applied onto the body,
wherein the applicator (2, 5, 5', 5") has a pivot bearing (14) which rotatably holds a rotatable applicator part (15, 16, 17) of the applicator (2, 5, 5', 5") which is designed to be applied onto the body to be treated, and
rotation of the rotatable applicator part (15, 16, 17) around an axis of rotation angled in relation to the direction of impact is possible,
**characterised in that** the mechanism for generating the impacts comprises a projectile and an pneumatic device for accelerating the projectile such that the projectile hits the applicator (2, 5, 5', 5"), thereby generating the impact, and wherein the applicator (2, 5, 5', 5") is held in the device by means of a positive locking arrangement such that it is locked against rotation, namely in respect of rotations around an axis parallel to the direction of impact.

2. The device according to claim 1, wherein the axis of rotation is perpendicular to the direction of impact.

3. The device according to claim 1 or 2, wherein the rotatable applicator part (15, 16, 17) is rotationally symmetrical in relation to the axis of rotation.

4. The device according to one of the preceding claims, wherein the rotatable applicator part (15, 16, 17) is mirror-symmetrical in relation to a plane perpendicular to the axis of rotation.

5. The device according to one of the preceding claims, wherein the rotatable applicator part (15, 17), through which in particular an axis rod defining the axis of rotation is inserted, is rotatably held on two opposite sides.

6. The device according to one of the preceding claims, wherein the rotatable applicator part (15, 16, 17) has edges rounded on the outside with a radius of curvature of at least 1 mm.

7. The device according to claim 3, also in conjunction with one of claims 4-6, wherein the rotatable applicator part (15) has a cylindrical barrel shape with a convex outer profile on a sectional plane containing the axis of rotation.

8. The device according to one of claims 1-6, wherein the rotatable applicator part (17) has at least two areas protruding radially in relation to the axis of rotation and designed for contact with the body to be treated.

9. The device according to one of the preceding claims, wherein the applicator (2, 5, 5', 5") in its rotatable part (15, 16, 17), which is designed to be applied onto the body, is made from metal, in particular aluminium or titanium, plastic, ceramic or wood.

10. The device according to one of the preceding claims, which is designed for an impact stroke in the direction of impact relative to the housing (9, 11, 12) of at least 1 mm.

11. The device according to one of the preceding claims, wherein the applicator (2, 5, 5', 5") is held in the housing (9, 11, 12) in a coupling (7, 8, 9, 10), whereby at least one distal applicator part (5) provided with the rotatable applicator part (15, 16, 17) can be removed from the housing (9, 11, 12) in the distal direction once the coupling (7, 8, 9, 10) has been released.

12. The device according to one of the preceding claims, wherein the applicator (2, 5, 5', 5") comprises at least two pieces, whereby a distal applicator part (5, 5', 5") is removable once the coupling (7, 8, 9, 10) has been released while a proximal applicator part (2) remains in the housing, and whereby the proximal applicator part (2) transfers the impact to the distal applicator part (5, 5', 5").

## Revendications

1. Appareil de traitement du corps humain ou animal, comportant:
- un applicateur (2, 5, 5', 5") destiné à être appliqué par voie externe sur le corps,
- un boîtier (9, 11, 12) dans lequel est retenu l'applicateur (2, 5, 5', 5"), et
- un mécanisme permettant la production d'impacts par l'applicateur (2, 5, 5', 5") relativement au boîtier (9, 11, 12) selon une direction d'impact de manière que les impacts puissent être couplés dans le corps lors de l'application;
ledit applicateur (2, 5, 5', 5") présentant un palier tournant (14) retenant en rotation une partie rotative (15, 16, 17) de l'applicateur (2, 5, 5', 5") destinée à être disposée sur le corps à traiter, et
une rotation de la partie d'applicateur rotative (15, 16, 17) étant possible sur un axe de rotation qui est à un certain angle par rapport à la direction d'impact,
**caractérisé en ce que** le mécanisme de production d'impacts présente un projectile et un dispositif pneumatique permettant d'accélérer le projectile de telle manière que le projectile frappe l'applicateur (2, 5, 5', 5") pour ainsi produire l'impact,
ledit applicateur (2, 5, 5', 5") étant retenu immobile en rotation dans l'appareil par complémentarité de forme, en ce qui concerne des rotations sur un axe parallèle à la direction d'impact.

2. Appareil selon la revendication 1, dans lequel l'axe de rotation est perpendiculaire à la direction d'impact.

3. Appareil selon la revendication 1 ou 2, dans lequel la partie d'applicateur rotative (15, 16, 17) est symétrique en rotation sur l'axe de rotation.

4. Appareil selon l'une des revendications précédentes, dans lequel la partie d'applicateur rotative (15, 16, 17) est en symétrie spéculaire par rapport à un plan perpendiculaire à l'axe de rotation.

5. Appareil selon l'une des revendications précédentes, dans lequel la partie d'applicateur rotative (15, 17) est retenue rotative sur deux côtés opposés, en étant notamment traversée par une tige axiale définissant l'axe de rotation.

6. Appareil selon l'une des revendications précédentes, dans lequel la partie d'applicateur rotative (15, 16, 17) présente des bords arrondis, sur l'extérieur, dont le rayon de courbure est d'au moins 1 mm.

7. Appareil selon la revendication 3, également en conjonction avec une des revendications 4 à 6, dans lequel la partie d'applicateur rotative (15) présente une forme de tonneau cylindrique à profil extérieur convexe dans un plan de coupe contenant l'axe de rotation.

8. Appareil selon l'une des revendications 1 à 6, dans lequel la partie d'applicateur rotative (17) présente au moins deux zones faisant radialement saillie par rapport à l'axe de rotation et conçues pour venir au contact du corps à traiter.

9. Appareil selon l'une des revendications précédentes, dans lequel l'applicateur (2, 5, 5', 5") se compose de métal, notamment d'aluminium ou de titane, de plastique, de céramique ou de bois, dans sa partie rotative (15, 16, 17) destinée à être appliquée.

10. Appareil selon l'une des revendications précédentes, conçu pour une course d'impact dans la direction d'impact par rapport au boîtier (9, 11, 12) qui est d'au moins 1 mm.

11. Appareil selon l'une des revendications précédentes, dans lequel l'applicateur (2, 5, 5', 5") est retenu dans le boîtier (9, 11, 12) dans un dispositif d'accouplement (7, 8, 9, 10), au moins une partie d'applicateur distale (5) présentant la partie d'applicateur rotative (15, 16, 17) pouvant être retirée du boîtier (9, 11, 12) dans la direction distale après que le dispositif d'accouplement (7, 8, 9, 10) a été débloqué.

12. Appareil selon l'une des revendications précédentes, dans lequel l'applicateur (2, 5, 5', 5") se compose d'au moins deux pièces, une partie d'applicateur distale (5, 5', 5") pouvant être retirée après qu'un dispositif d'accouplement (7, 8, 9, 10) a été débloqué tandis qu'une partie d'applicateur proximale (2) demeure dans le boîtier, ladite partie d'applicateur proximale (2) transmettant l'impact à la partie d'applicateur distale (5, 5', 5").
